# EUROPÄISCHE PATENTSCHRIFT

(11) **EP 0 996 426 B1**
(45) Veröffentlichungstag und Bekanntmachung des Hinweises auf die Patenterteilung: **07.03.2007**
(21) Anmeldenummer: 98936136.5
(22) Anmeldetag: 05.06.1998
(51) Int. Cl.: A61K 9/00, A61K 9/20

(54) **MULTIPHASENSYSTEM**
MULTIPHASE SYSTEM
SYSTEME MULTIPHASES

(30) Priorität: 05.06.1997 DE 19724784; 13.03.1998 DE 19811951
(43) Veröffentlichungstag der Anmeldung: 03.05.2000
(73) Patentinhaber: Bodmeier, Roland, 14163 Berlin (DE)
(72) Erfinder: Bodmeier, Roland, 14163 Berlin (DE)
(74) Vertreter: Schubert, Klemens
(86) Internationale Anmeldenummer: PCT/DE1998/001589
(87) Internationale Veröffentlichungsnummer: WO 1998/055100

(56) Entgegenhaltungen:
- WO-A-96/39995
- DE-C- 4 122 591
- US-A- 4 938 763
- US-A- 5 278 202
- US-A- 5 340 849
- US-A- 5 733 950
- US-A- 5 747 058

## Beschreibung

Die vorliegende Erfindung betrifft eine Zubereitung, umfassend eine Trägerphase und mindestens eine mit der Trägerphase nicht oder teilweise nicht mischbare.weitere Phase, wobei sich bei der Veränderung der Umgebungsbedingungen die Viskosität der Trägerphase verändert.

Für die Behandlung bestimmter Krankheiten kann der Arzneistoff als Depotform parenteral appliziert werden. Dazu bieten sich neben klassischen Arzneiformen, wie z._B. öligen Suspensionen, moderne Arzneiformen auf der Basis biokompatibler/bioabbaubarer Polymere an. Da die polymeren Träger fest sind, werden diese in entsprechende Implantate (Ein-Körper-System) oder Mikropartikel (multipartikuläre Systeme) verarbeitet und dann in den Körper durch Implantation oder Injektion eingebracht.

Zur Implantatherstellung wird der Arzneistoff mit dem Träger (z.B. ein Polymer) gemischt und dann in die gewünschte Implantatform (z.B. Zylinder, Pellet, Film, Faser), z.B. durch Extrusion oder Verpressen bei erhöhten Temperaturen gebracht. Solche festen Implantate werden dann meist durch einen chirurgischen Eingriff oder durch Hohlnadeln mit großem Durchmesser in den Körper eingebracht.

Um solche chirurgischen Eingriffe bei Implantaten, die von Patienten meist unerwünscht sind, zu umgehen, können arzneistoffhaltige Mikropartikel verwendet werden. Suspensionen dieser Partikel können aus einer Spritze durch eine Injektionsnadel injiziert werden. Diese Mikropartikel werden außerhalb des Körpers durch verschiedene Verfahren wie z.B. Lösungsmittelverdampfungs- ("Solvent evaporation"-), organische Phasenseparations- oder Sprühtrocknungs-Verfahren hergestellt. In der zur Herstellung bioabbaubarer Mikropartikel häufig verwendeten Solvent-Evaporations-Methode wird z.B. ein Arzneistoff in einer Lösung eines bioabbaubaren Polymeren, wie z.B. Polymilchsäure, in einem mit Wasser nicht mischbaren Lösungsmittel (z.B. Dichlormethan) gelöst oder dispergiert. Diese arzneistoffhaltige Polymerphase wird dann in eine äußere wäßrige Phase zur Bildung wirkstoffhaltiger Polymertröpfchen emulgiert. Die Mikropartikel werden nach Verdampfen des Lösungsmittels durch Erhärten des Polymeren erhalten und dann von der wäßrigen Phase z.B. durch Filtration abgetrennt und getrocknet.

Handelspräparate bioabbaubarer Mikropartikel (z.B. Decapeptyl, Enantone) bestehen aus einem Trockenpulver der Mikropartikel und einem wäßrigen Suspensionsvehikel. Aufgrund der hydrolytischen Instabilität der bioabbaubaren Polymeren werden die Mikropartikel und das wäßrige Suspensionsvehikel getrennt, z.B. in Zweikammerspritzen oder in zwei Ampullen, aufbewahrt. Die Mikropartikel werden dann unmittelbar vor der Anwendung in dem wäßrigen Suspensionsvehikel suspendiert und dann injiziert.

Die Herstellung dieser bioabbaubaren Partikelpräparate ist sehr aufwendig und muß unter sterilen oder aseptischen Bedingungen erfolgen. Weiterhin sind die meisten Mikroverkapselungsverfahren nur schwer oder gar nicht auf den Produktionsmaßstab zu übertragen und abhängig von vielen Prozeß- und Formulierungsvariablen. Ferner kann die Suspendierung der Mikropartikel mit nachfolgender Injektion mit Schwierigkeiten verbunden sein (z.B. Agglomeration, Mikropartikelrückstände in der Spritze, Verstopfen der Kanüle, etc.).

Neben den wasserunlöslichen Polymilchsäurederivaten und anderen wasserunlöslichen Polymeren können auch hydrophile Polymere als Trägermaterialien für Mikropartikel oder Implantate eingesetzt werden. Mikropartikel aus hydrophilen Polymeren (z.B. Polysaccharide wie Alginate oder Chitosan, Cellulosederivate, Protein-(Kollagen)-derivate) können z.B. durch Sprühtrockung oder w/o-Emulsionsverfahren hergestellt werden, wobei die wirkstoffhaltige wäßrige Polymerlösung entweder sprühgetrocknet oder in eine äußere, ölige Phase emulgiert wird und die Partikel nach Entfernung des Wassers, Waschens, Filtration und Trocknung erhalten werden. Ähnlich wie die Verfahren zur Herstellung von Polymilchsäure-Mikropartikel sind auch die Mikroverkapselungsverfahren mit hydrophilen Polymeren sehr aufwendig.

Um die Probleme bei der Herstellung und Anwendung von Implantaten und Mikropartikeln zu vermeiden, wurde eine Zubereitung bestehend aus einer arzneistoffhaltigen Polymerlösung entwickelt (US-PS 4,938,763). Dabei wird eine Polymilchsäure(derivat)lösung in den Körper, z.B. intramuskulär oder subcutan injiziert, ein Implantat bildet sich in-situ durch Ausfällen des Polymeren im Gewebe. Das Implantat wird also nicht außerhalb sondern erst innerhalb des Körpers gebildet. Die Polymerlösung muß durch eine Nadel injizierbar sein, darf also nicht zu viskos sein. Der Polymergehalt ist damit in erster Linie durch die Viskosität und nicht durch die Löslichkeit des Polymeren limitiert. Auch während der Injektion der Polymerlösung kann durch Ausfällung des Polymeren die Injektion der verbleibenden Polymerlösung negativ beeinflußt werden. Nachteile dieser Methode sind ferner die Verwendung hoher Lösungsmittelanteile mit entsprechender Toxizität oder Verträglichkeitsproblemen und, nach Injektion in das Weichgewebe, die etwas unkontrollierte Verhärtung des Polymeren mit nicht genau definierter Oberfläche des Implantats. Dies kann zu irreproduzierbaren Freisetzungsprofilen führen. Ferner kann der Wirkstoff vor der Verhärtung der Polymerlösung rasch freigesetzt werden. Dieser sogenannte "Burst-Effekt" ist meist unerwünscht.

Ferner wurden einige Systeme entwickelt, bei denen eine Verfestigung/Viskositätserhöhung wirkstoffhaltiger Polymerlösungen nach Applikation/Injektion in den Körper nicht durch Lösungsmitteldiffusion, sondern in erster Linie durch Temperatur- oder pH-Änderung oder durch bestimmte Substanzen (z.B. Ionen) hervorgerufen wird. Diese Systeme haben meist die gleichen Nachteile wie das im vorhergehenden Paragraphen beschriebene System.

Aufgabe der Erfindung ist die Schaffung von Zubereitungen, die einfach herzustellen sind, und die Vermeidung von Problemen, die bei der Entwicklung und Anwendung von Mikropartikeln und Implantaten, auch der beschriebenen in-situ Implantate, auftreten.

Diese Aufgabe wird durch eine Zubereitung gemäß Anspruch 1 gelöst.

Erfindungsgemäß ist vorgesehen, daß die Zubereitung aus einer Dispersion einer inneren Trägerphase und einer mit der Trägerphase nicht mischbaren oder teilweise nicht mischbaren äußeren zweiten Phase besteht, sich nach Veränderung der Umgebungsbedingungen Partikel bilden, sich nach Veränderung der Umgebungsbedingungen ein Implantat bildet, die Veränderung der Umgebungsbedingungen eine Konzentrierung des Trägermaterials in der Trägerphase hervorruft, die Veränderung der Umgebungsbedingungen eine Ausfällung des Trägermaterials hervorruft, die Veränderung der Umgebungsbedingungen eine Wegdiffusion des Lösungsmittels aus der Trägerphase hervorruft, die Veränderung der Umgebungsbedingungen durch Kontakt der Zubereitung mit Körperbestandteilen hervorgerufen ist, die Veränderung der Umgebungsbedingungen eine Temperaturänderung ist, die Veränderung der Umgebungsbedingungen eine Temperaturerhöhung ist, die Veränderung der Umgebungsbedingungen eine pH-Änderung ist, die Veränderung der Umgebungsbedingungen durch am Applikationsort vorhandene Substanzen hervorgerufen ist, die Veränderung der Umgebungsbedingungen eine Veränderung der Ionenstärke oder - art ist, die Veränderung der Umgebungsbedingungen eine Kombination von zwei oder mehreren Umgebungsbedingungen ist, sie durch Hochdruckhomogenisierung hergestellt ist, die Teilchengröße der Trägerphase überwiegend kleiner als 100 µm ist, vorzugsweise kleiner als 20 µm ist, die Teilchengröße der Trägerphase überwiegend im kolloidalen Bereich liegt, sie kurz vor der Applikation hergestellt wird, die Trägerphase und die zweite Phase entweder getrennt oder in Kontakt, aber in nicht oder in nur teilweise dispergiertem Zustand aufbewahrt sind, das Trägermaterial ein wasserunlösliches Polymer ist, das Trägermaterial ein in wäßrigen Flüssigkeiten lösliches Polymer ist, das Trägermaterial ein wasserlösliches Polymer ist, das Trägermaterial ein Cellulose- oder Acrylatderivat ist, das Trägermaterial ein biokompatibles und/oder bioabbaubares Polymer ist, das Trägermaterial Polylaktid oder ein Polylaktid-derivat ist, das Trägermaterial ein Polysaccharid ist, die Trägerphase aus einer Kombination von Trägermaterialien besteht, das Trägermaterial in der Trägerphase gelöst und/oder dispergiert vorliegt, die Trägerphase aus einer Trägerschmelze besteht. Erfindungsgemäß ist ferner vorgesehen, daß die erfindungsgemäße Zubereitung weiterhin mindestens einen Wirkstoff umfaßt, der Wirkstoff ein Arzneistoff ist, der Wirkstoff ein Peptid- oder Proteinarzneistoff ist, der Wirkstoff in der Trägerphase gelöst, dispergiert, suspendiert oder emulgiert ist, der Wirkstoff zusätzlich oder ausschließlich in den anderen Phasen vorliegt.

Erfindungsgemäß ist es ferner, daß die Trägerphase Wasser oder ein organisches Lösemittel, wie Ethanol, Aceton, Butanol, Ethylformat, Pentanol, n- oder i-Propanol, Tetrahydrofuran, Triethylcitrat, Triacetin, Propylenglykol, Glycerol, Polyethylenglykol, Aceton, Ethylacetat, Methylacetat, Dimethylformamid, Dimethylsulfoxid, Dimethylacetamid, 2-Pyrrolidon, N-Methyl-2-Pyrrolidon oder eine Mischung aus zwei oder mehreren der oben genannten Lösemittel enthält, die zweite Phase mit wäßrigen Flüssigkeiten vollständig, teilweise oder nicht mischbar ist, die Zubereitung eine dritte Phase umfaßt, die Trägerphase und die zweite Phase mit der weiteren dritten Phase gemischt sind, die zweite Phase mit der dritten Phase nicht oder nur teilweise mischbar ist, die dritte Phase vollständig, teilweise oder nicht mit wäßrigen Flüssigkeiten mischbar ist, die zweite Phase natürliche, halbsynthetische oder synthetische Fette, Öle oder Wachse, wie Baumwollensaatöl, Sojaöl, Saffloröl, hydriertes Erdnußöl, Olivenöl, Rizinusöl, Triglyceridgemische (wie Miglyol), Silikonöl, Isopropylmyristat, Ethyloleat, Paraffine, Glycerol, Propylenglycol oder Polyethylenglycol oder Mischungen aus zwei oder mehreren der oben genannten Stoffe umfaßt, die zweite Phase mit wäßrigen Flüssigkeiten mischbar ist.

Es ist ferner vorgesehen, daß die Zubereitung viskositätserhöhende Substanzen enthält, sie thixotropes Fließverhalten aufweist, sie Stabilisatoren enthält, sie Freigaberegulatoren enthält, sie Substanzen enthält, welche die Verweildauer am Applikationsort verändert, sie bioadhäsive Materialien enthält, sie Penetrationsverbesserer enthält.

Die erfindungsgemäße Zubereitung bewirkt, daß der Wirkstoff am Applikationsort verzögert freigegeben wird oder daß eine anfängliche, rasche Freigabe des Wirkstoffes am Applikationsort vermieden wird.

Bei der erfindungsgemäßen Zubereitung ist der Applikationsort lebendes oder totes biologisches Gewebe.

Die Anwendung der erfindungsgemäßen Zubereitung ist zur zur parenteralen, peroralen, subcutanen, rectalen, buccalen, vaginalen, nasalen, lokalen, sublingualen, peridontalen oder transdermalen Anwendung vorgesehen. Dabei wird die Zubereitung erfindungsgemäß in Körperöffnungen eingebracht.

Die Zubereitung kann erfindungsgemäß in Form einer Kapsel vorliegen.

Eine erfindungsgemäße Zubereitung ist z.B. eine Dispersion bestehend aus einer inneren wirkstoffhaltigen Trägerphase und einer äußeren, zweiten Phase.

Gegenstand der vorliegenden Erfindung Verfahren zur Herstellung einer erfindungsgemäßen Zubereitung, wobei man eine Mischung einer flüssigen Trägerphase und einer zweiten Phase herstellt und diese Mischung in einen lebenden Körper einbringt und Partikel bildet.

Erfindungsgemäß ist es, daß die Trägerphase mit der zweiten Phase nicht oder nur teilweise mischbar ist und eine Dispersion bildet, daß die Trägerphase die innere Phase dieser Dispersion ist und Partikel bildet, daß man die Zubereitung durch Hochdruckhomogenisierung herstellt, daß man die Zubereitung unter Erwärmen herstellt, daß die Teilchengröße der Trägerphase kleiner als 200 µm ist, daß die Teilchengröße der Trägerphase im kolloidalen Bereich liegt, daß man die Zubereitung kurz vor dem Einbringen in den Körper aus der Trägerphase und der äußeren Phase herstellt, daß man die Trägerphase und die äußere Phase getrennt voneinander aufbewahrt, daß man die Trägerphase und die äußere Phase in einer Zweikammerspritze aufbewahrt, daß man die Trägerphase und die äußere Phase in Kontakt, aber in nicht oder nur teilweise dispergiertem Zustand aufbewahrt, daß der Träger in der Trägerphase gelöst vorliegt, daß das Trägermaterial ein wasserunlösliches Polymer ist, daß das Trägermaterial ein Cellulosederivat ist, daß das Trägermaterial ein Acrylatderivat ist, daß das Trägermaterial ein biokompatibles oder ein bioabbaubares Polymer ist, daß das Trägermaterial ein Polylaktid oder ein Polylaktid-Glykolidcopolymer ist, daß das Lösungsmittel für den Träger ein organisches Lösungsmittel ist, daß man ein Lösungsmittelgemisch verwendet, daß das Lösungsmittel mit Wasser mischbar ist oder daß die zweite Phase mit Wasser nicht oder nur teilweise mischbar ist.

Erfindungsgemäß ist es ferner, daß die zweite Phase natürliche, halbsynthetische oder synthetische Fette, Öle oder Wachse enthält, daß die zweite Phase mit Wasser mischbar ist, daß man übliche Hilfsstoffe zusetzt, daß man der Zubereitung viskositätserhöhende Substanzen zusetzt, daß die Zubereitung thixotropes Fließverhalten zeigt, daß man der Zubereitung Stabilisatoren zusetzt, daß man der Zubereitung Freigaberegulatoren zusetzt.

Erfindungsgemäß bevorzugt ist ein Verfahren, wobei man der Zubereitung Wirkstoffe beigibt, man den Wirkstoff in der Trägerphase löst, dispergiert, suspendiert oder emulgiert, der Wirkstoff auch in der äußeren Phase vorliegt, der Wirkstoff ein Peptid- oder Proteinarzneistoff ist. Partikel sind erhältlich durch das Einbringen einer erfindungsgemäßen Zubereitung am Applikationsort. Die Partikel werden daher nicht separat durch aufwendige Mikroverkapselungsverfahren hergestellt und kurz vor der Anwendung suspendiert und in den Körper eingebracht, sondern die Partikel bilden sich in-situ nach Einbringen der Zubereitung in den Körper aus der inneren Trägerphase. Der Sammelbegriff "Partikel" wird für Pellets, Mikro- und Nanopartikel verwendet. Partikel können auch aus Agglomeraten kleinerer Partikel bestehen.

Ein Implantat wird vorteilhafterweise in ein lebendes Gewebe durch eine Injektion eingebracht, wobei durch die Viskositätsänderung der Zubereitung ein Implantat gebildet wird. Dieses Implantat enthält dann vorteilhafterweise beispielsweise Arzneistoffe, welche dann über einen längeren Zeitraum hin an das Gewebe abgegeben werden. Ein Implantat kann sich z.B. durch teilweises Zusammenfließen der Trägerphase bilden.

Erfindungsgemäß ist es, daß die Veränderung der Umgebungsbedingungen eine Konzentrierung des Trägermaterials in der Trägerphase, eine Ausfällung des Trägermaterials, eine Wegdiffusion des Lösungsmittels aus der Trägerphase, ein Kontakt der Trägerphase mit Umgebungsbestandteilen, eine Temperaturänderung, eine Änderung des pH-Wertes, eine Änderung der Ionenstärke, das Einbringen der Zubereitung am Applikationsort oder eine Kombination von zwei oder mehrerer der genannten Umgebungsbedingungen ist.

Im Falle einer Temperaturerhöhung kann zum Beispiel die innere Trägerphase nach Einbringen in den Körper durch Erwärmen auf Körpertemperatur von einem Sol- in den Gel-Zustand umgewandelt und damit verfestigt werden. Dabei können auch Hilfsstoffe eingearbeitet werden, die die Verfestigung beschleunigen oder verzögern. Die Zubereitung kann dazu auch kurz vor der Applikation erwärmt werden. Ferner kann die Viskositätsveränderung der Trägerphase durch pH-Änderung oder durch am Applikationsort vorhandenen Substanzen, z.B. Ionen, hervorgerufen werden.

Zum Beispiel kann eine wirkstoffhaltige Dispersion, bestehend aus einer inneren Trägerphase und einer zweiten, äußeren Phase (z.B. ein Öl), hergestellt und in den Körper eingebracht werden. Es kommt dann zu einer Verfestigung der inneren Phase, z.B. durch Lösungsmitteldiffusion in die Umgebung oder Eindiffusion von Körperflüssigkeiten, oder durch Änderung der Temperatur, des pH-Wertes oder der Ionenstärke. Die Dispersion kann z.B. im Falle von bioabbaubaren Polymeren i.m. oder s.c. injiziert werden, im Falle peroraler Anwendung kann die flüssige Dispersion in Kapseln abgefüllt werden. Im Kontakt mit Körperflüssigkeiten kann sich die innere Phase verfestigen und z.B. Partikel bilden.

Die Trägerphase wird bevorzugt in überwiegend flüssiger/halbfester und nicht in fester Form wie im Falle von Mikropartikeln oder Implantaten in den Körper eingebracht. Die trägerhaltige Phase der Zubereitung verfestigt sich dann im Körper, der Wirkstoff kann dann verzögert freigesetzt werden. Bei dieser Methode handelt es sich praktisch um die Herstellung einer Dispersion/Emulsion. Die aufwendige Herstellung von Mikropartikeln oder Implantaten und die Resuspendierung der Partikel vor der Applikation entfallen.

Die Zubereitungen können durch dem Fachmann bekannte Verfahren aus der Trägerphase, der zweiten und eventuell der dritten Phase hergestellt werden. Die Herstellung flüssiger oder halbfester Zubereitungen fällt in den Bereich klassischer pharmazeutisch-technologischer Verfahrenstechniken. Die Teilchengröße und Teilchengrößenverteilung der inneren Phase und damit auch indirekt der verfestigten Partikel kann z.B. besonders durch die Art und Intensität des Emulgierverfahrens beeinflußt werden. Neben dem Emulgierverfahren beeinflussen natürlich auch noch andere Parameter die Teilchengröße, z.B. die Auswahl des Emulgators bzw. des Emulgatorkomplexes, und die Viskosität der inneren und äußeren Phase. Dispersionen mit kleinerer Teilchengröße (z.B. kolloidaler Teilchengrößenbereich) können z.B. durch Hochdruckhomogenisierung erhalten werden. Die Zubereitungen können auch unter Erwärmen oder durch Phasenumkehr hergestellt werden.

Die Zubereitung kann auch erst kurz vor der Anwendung hergestellt werden. Die einzelnen Phasen oder auch einzelne Bestandteile (z.B. Wirkstoff) können voneinander getrennt oder teilweise getrennt aufbewahrt werden, z.B. in einer Zweikammerspritze oder in speziellen Behältnissen, die ein effizientes Mischen der Phasen erlauben. Dies ist vor allem bei Systemen mit physikalischen oder chemischen Stabilitätsproblemen vorteilhaft.

Das Trägermaterial der Trägerphase ist ein Cellulosederivat (z.B. Celluloseacetat, Ethylcellulose, Celluloseacetatphthalat), Acrylatderivate (z.B. Eudragite, Poly(methylmethacrylat), Cyanoacrylate) und Polymere wie Polyanhydride, Polyester, Polyorthoester, Polyurethane, Polycarbonate, Polyphosphazene und Polyacetale. Wichtig dabei sind Polyester wie Polylaktid, Polyglykolid, Polycaprolacton, Polyhydroxybutyrat-oder valerat. Ferner können auch Polysaccharide wie Na Alginate, Chitosan oder Chitin verwendet werden. Es können selbstverständlich auch Kombinationen der Trägermaterialien oder Co- oder Terpolymere verwendet werden.

Erfindungsgemäß ist, daß das Trägermaterial in der Trägerphase gelöst, geschmolzen oder auch dispergiert vorliegt. Meist ist das Trägermaterial für die durch Veränderung der Umgebungsbedingungen hervorgerufene Viskositätsveränderung verantwortlich.

Erfindungsgemäß kann auch vorgesehen sein, daß das Trägermaterial selbst die Trägerphase ist. Dabei hat das Trägermaterial selbst eine halbfeste Konsistenz. Ein Zusatz von Lösemitteln unterbleibt dann erfindungsgemäß.

Die maximal verwendbare Trägerkonzentration hängt dabei überwiegend von der Viskosität der Trägerphase und der Intensität des Dispergiergerätes ab. Obwohl die Trägerphase alleine, z.B. zur parenteralen Injektion, nicht ausreichend fließfähig ist, kann durch ihre erfindungsgemäße Einarbeitung in die äußere fließfähige Phase eine injizierbare Zubereitung erhalten werden. Es können daher im Vergleich zu injizierbaren Polymerlösungen wesentlich höher konzentrierte Polymerlösungen und damit weniger Lösungsmittel verwendet werden.

Zu den Wirkstoffen zählen nieder- und höhermolekulare Arzneistoffe (z.B. auch Peptide, Proteine, Oligonucleotide) zur human- und veterinärmedizinischen Anwendung und Substanzen, die in der Landwirtschaft, im Haushalt, in der Nahrungsmittel-, kosmetischen und chemischen Industrie und anderen Industriezweigen genutzt werden. Vorteilhafterweise ist der Wirkstoff in der Trägerphase gelöst. Erfindungsgemäß bevorzugt ist aber auch, daß der Wirkstoff in der Trägerphase dispergiert, suspendiert oder emulgiert ist. Erfindungsgemäß vorteilhaft ist es ferner, daß der Wirkstoff zusätzlich oder ausschließlich in den anderen Phasen vorliegt. Zum Beispiel kann ein Teil des Wirkstoffes der äußeren Phase, z.B. zum Erzielen einer Initialdosis, beigegeben werden. Selbstverständlich können auch Kombinationen von Wirkstoffen verwendet werden.

Vorteilhaft ist es ferner, daß die Trägerphase Wasser oder ein organisches Lösemittel, wie Ethanol, Aceton, Butanol, Ethylformat, Pentanol, n- oder i-Propanol, Tetrahydrofuran, Triethylcitrat, Triacetin, Propylenglykol, Glycerol, Polyethylenglykol, Aceton, Ethylacetat, Methylacetat, Dimethylformamid, Dimethylsulfoxid, Dimethylacetamid, 2-Pyrrolidon, N-Methyl-2-Pyrrolidon oder eine Mischung aus zwei oder mehreren der oben genannten Lösemittel umfaßt. Es bieten sich vor allem auch Lösungsmittel aus der "Draft guideline of the international conference on harmonization on impurities - residual solvents" an. Es können auch Lösungsmittelgemische verwendet werden, ausgewählt z.B. nach Lösungsmittelqualität für das Polymer oder Mischbarkeit mit wäßrigen und öligen Phasen. Durch die Lösungsmittelauswahl kann z.B. die Mischbarkeit der Trägerflüssigkeit mit Körperflüssigkeiten, der äußeren Phase und die Verfestigung der Trägerphase beeinflusst werden. Die Viskosität der Trägerphase kann durch den Träger (z.B. Molmasse, Konzentration etc.) und auch das Lösungsmittel beeinflußt werden. Der Verfestigungsgrad kann durch die Auswahl des Polymeren, des Lösungsmittels und der äußeren Phase beeinflußt werden.

Erfindungsgemäß bevorzugt ist es, daß die zweite Phase vollständig, teilweise oder nicht mit wäßrigen Flüssigkeiten mischbar ist.

Erfindungsgemäß vorteilhaft ist es, daß die zweite Phase natürliche, halbsynthetische oder synthetische Fette, Öle oder Wachse, wie Baumwollensaatöl, Sojaöl, Saffloröl, hydriertes Erdnußöl, Olivenöl, Rizinusöl, Triglyceridgemische (wie Miglyol), Silikonöl, Isopropylmyristat, Ethyloleat, Paraffine, Glycerol, Propylenglycol oder Polyethylenglycol oder Mischungen aus zwei oder mehreren der oben genannten Stoffe umfaßt.

Erfindungsgemäß ist es, daß die Zubereitung eine dritte Phase umfaßt. Bevorzugt ist es dabei, daß die Trägerphase und die zweite Phase mit der weiteren dritten Phase gemischt sind. Erfindungsgemäß bevorzugt ist es ferner, daß die zweite Phase mit der dritten Phase nicht oder nur teilweise mischbar ist. Weiterhin erfindungsgemäß bevorzugt ist es, daß die dritte Phase vollständig, teilweise oder nicht mit wäßrigen Flüssigkeiten mischbar ist. Verschiedene Variationen von multidispersen Systemen sind daher möglich.

Insbesondere bevorzugt sind erfindungsgemäße Zubereitungen, bei denen mindestens eine der Phasen viskositätserhöhende Substanzen, Stabilisatoren, Freigaberegulatoren, die Verweildauer am Anwendungsort verlängernde oder verkürzende Substanzen, bioadhäsive Materialien, Penetrationsverbesserer, die Wirkstofffreigabe verzögernde, beschleunigende, verhindernde oder eine anfängliche, rasche Freigabe des Wirkstoffes vermeidende Substanzen oder beliebige Kombinationen der oben genannten Substanzen enthält.

Der Zubereitung können viskositätserhöhende Stoffe, z.B. Fettsäuresalze mehrwertiger Kationen, Polymere, Siliciumderivate oder höherschmelzende Lipide beigegeben werden. Das Fließverhalten der äußeren Phase kann auch durch Zusätze verändert werden, z.B. kann eine Phase gebildet werden, bei der es während der Injektion zur einer Verringerung und im Ruhestand zu einer Erhöhung der Viskosität kommt (thixotropes Fließverhalten).

Zur Herstellung der Dispersion können Stabilisatoren, wie z.B. Emulgatoren notwendig sein. Zu den Emulgatoren zählen unter anderem Polyethylenglykol-fettsäureester, -fettsäureether, -sorbitanfettsäureester, Sorbitanfettsäureester, Partialfettsäureester mehrwertiger Alkohole oder Zucker, Lecithine und Poloxamere.

Die Verweildauer der Zubereitung am Applikationsort kann durch entsprechende Substanzen, z.B. bioadhäsive Materialien verlängert werden. Diese Substanzen können sowohl der Trägerphase und/oder den anderen Phasen zugesetzt werden. Ferner können Penetrationsverbesserer zugegeben werden, die Wirkstoffresorption verbessern.

Die Wirkstofffreisetzung kann unter anderem durch den Dispersitätsgrad, die Wirkstoffbeladung, das Polymer, die Polymerkonzentration, und die Molmasse des Polymeren beeinflusst werden. Ferner können auch Freigaberegulatoren, wie z.B. hydrophile oder lipophile Stoffe anorganischer, organischer oder polymerer Natur mit eingearbeitet werden. Ein besonderer Vorteil der Zubereitung ist, daß im Vergleich zu wirkstoffhaltigen Polymerphasen, die in-situ ein Implantat bilden, eine anfängliche, rasche Freigabe des Wirkstoffes am Applikationsort vermieden wird. Die wirkstoffhaltige Trägerphase stellt die innere Phase der Zubereitung dar und ist daher nach der Applikation überwiegend nicht sofort in Kontakt mit dem umgebenden Körper.

Erfindungsgemäß bevorzugt ist es, daß der Applikationsort lebendes oder totes biologisches Gewebe ist.

Die erfindungsgemäße Zubereitung ist vorteilhafterweise zur parenteralen, peroralen, subcutanen, rectalen, buccalen, vaginalen, lokalen, sublingualen, peridontalen oder transdermalen Anwendung bestimmt.

Auch bei der peroralen Applikation gewinnen multipartikuläre Systeme in Form von Pellets oder Mikro-/Nanopartikel im Vergleich zu sogenannten "single-unit"-Systemen wie Tabletten oder Kapseln immer mehr an Bedeutung. Neben der parenteralen Applikation können die Zubereitungen daher vor allem auch peroral, aber auch in verschiedenen Körperöffnungen (z.B. rektal, vaginal oder peridontal) angewendet werden. Zur Herstellung der fertigen Darreichungsform können die erfindungsgemäßen Zubereitungen z.B. in eine Spritze oder in eine Ampulle abgefüllt werden oder in eine Kapsel gefüllt werden.

Neben der Anwendung der erfindungsgemäßen Zubereitungen auf pharmazeutischem Gebiet, können die erfindungsgemäßen Zubereitungen auch auf anderen Gebieten verwendet werden, wo ein Wirkstoff über einen längeren Zeitraum in vorherbestimmter Weise an die Umgebung abgegeben werden soll. Derartige Systeme können beispielsweise Langzeitdünger, Schädlingsbekämpfungsmittel oder Lockstoffe für die Pflege von Pflanzen sein. Der Begriff Umgebungsbedingung und Applikationsort ist erfindungsgemäß weit gefaßt und nicht auf die Anwendung im medizinischen Bereich beschränkt.

Durch die nachfolgenden Beispiele wird die Erfindung erläutert, soll dadurch jedoch nicht eingeschränkt werden.

### Beispiel 1

Poly(d,1-lactid) (Resomer-203, Boehringer Ingelheim) wird in Dimethylsulfoxid (DMSO) und PEG 400 und Tween 80 gelöst. Aluminium stearat (2%) wird in Erdnußöl unter Erwärmen eingearbeitet, anschließend wird die Temperatur gesenkt und Span 80 zugemischt. Diese Polymerphase wird in die zweite Phase zur Bildung einer Emulsion einemulgiert. Alternativ zu DMSO kann auch N-Methyl-2-Pyrrolidon als Lösungsmittel verwendet werden.

### Beispiel 2

Poly(d,1-lactid) (Resomer-203, Boehringer Ingelheim) wird in Triethylcitrat und Tween 80 (3%, bezogen auf die innere Phase) gelöst. Diese Polymerphase wird in Glycerol zur Bildung der Dispersion einemulgiert.

### Beispiel 3

Poly(oxyethylen-oxypropylen) (Lutrol F 127 (BASF)) wird in Wasser gelöst (>20%m/m) und in die äußere Erdnußölphase emulgiert. Bei Temperaturerhöhung auf 37 °C kommt es zu einer Erhöhung der Viskosität der inneren Phase.

### Beispiel 4

Chitosan wird in saurem, wäßrigem Milieu oder ein Chitosansalz (z.B. Chitosanglutamat) wird in Wasser gelöst. Diese Lösung wird in die äußere Ölphase emulgiert. In Kontakt mit Puffer pH 7,4 kommt es zum Ausfällen von Chitosan.

## Patentansprüche

1. Wirkstoffhaltige Zubereitung, die bei Einbringen in einen Körper Partikel bildet, erhältlich durch Herstellen einer Dispersion aus einer flüssigen inneren polymerhaltigen Trägerphase und einer nicht mischbaren oder teilweise nicht mischbaren zweiten äußeren Phase und nachfolgender Veränderung der Umgebungsbedingungen, wobei sich die Viskosität der Trägerphase verändert, und wobei das Polymer ausgewählt ist aus einem Cellulosederivat, einem Polyacrylatderivat, einem Polysaccharid, einem Polyanhydrid, einem Polyester, einem Polyorthoester, einem Polyurethan, einem Polycarbonat, einem Polyphosphazen und einem Polyacetal oder einer Kombination von genannten Polymeren.

2. Zubereitung gemäß Anspruch 1, **dadurch gekennzeichnet, dass** die Veränderung der Umgebungsbedingungen eine Konzentrierung des Trägermaterials in der Trägerphase, eine Ausfällung des Trägermaterials, eine Wegdiffusion des Lösungsmittels hervorruft, ein Inkontaktbringen mit Körperbestandteilen, eine Temperaturänderung, eine pH-Änderung, eine Veränderung der Ionenstärke oder - art, ein Inkontaktbringen mit am Applikationsort vorhandenen Substanzen oder eine Kombination von zwei oder mehreren der genannten Veränderungen der Umgebungsbedingungen ist.

3. Zubereitung nach einem oder mehreren der vorhergehenden Ansprüche, **dadurch gekennzeichnet, dass** sie durch Hochdruckhomogenisierung erhältlich ist.

4. Zubereitung nach einem oder mehreren der vorhergehenden Ansprüche, **dadurch gekennzeichnet, dass** die Teilchengröße der Trägerphase überwiegend kleiner als 100 µm, vorzugsweise kleiner als 20 µm ist.

5. Zubereitung nach einem oder mehreren der vorhergehenden Ansprüche, **dadurch gekennzeichnet, dass** die Teilchengröße der Trägerphase überwiegend im kolloidalen Bereich liegt.

6. Zubereitung nach einem oder mehreren der vorhergehenden Ansprüche, **dadurch gekennzeichnet, dass** sie kurz vor der Applikation hergestellt wird.

7. Zubereitung nach einem oder mehreren der vorhergehenden Ansprüche, **dadurch gekennzeichnet, dass** die Trägerphase und die zweite Phase entweder getrennt oder in Kontakt, aber in nicht oder in nur teilweise dispergiertem Zustand aufbewahrt sind.

8. Zubereitung nach einem oder mehreren der vorhergehenden Ansprüche, **dadurch gekennzeichnet, dass** das Trägermaterial in der Trägerphase gelöst und/oder dispergiert vorliegt oder die Trägerphase selbst bildet.

9. Zubereitung nach einem oder mehreren der vorhergehenden Ansprüche, **dadurch gekennzeichnet, dass** der Wirkstoff ein Arzneistoff, ein Peptid- oder Proteinarzneistoff ist.

10. Zubereitung nach einem oder mehreren der vorhergehenden Ansprüche, **dadurch gekennzeichnet, dass** der Wirkstoff in der Trägerphase gelöst, dispergiert, suspendiert oder emulgiert ist oder zusätzlich oder ausschließlich in den anderen Phasen vorliegt.

11. Zubereitung nach mindestens einem der voranstehenden Ansprüche, **dadurch gekennzeichnet, dass** die Trägerphase Wasser oder ein organisches Lösemittel, wie Ethanol, Aceton, Butanol, Ethylformat, Pentanol, n- oder i-Propanol, Tetrahydrofuran, Triethylcitrat, Triacetin, Propylenglykol, Glycerol, Polyethylenglykol, Aceton, Ethylacetat, Methylacetat, Dimethylformamid, Dimethylsulfoxid, Dimethylacetamid, 2-Pyrrolidon, N-Methyl-2-Pyrrolidon oder eine Mischung aus zwei oder mehreren der oben genannten Lösemittel enthält.

12. Zubereitung nach einem oder mehreren der vorhergehenden Ansprüche, **dadurch gekennzeichnet, dass** die zweite Phase mit wäßrigen Flüssigkeiten vollständig, teilweise oder nicht mischbar ist.

13. Zubereitung nach einem oder mehreren der vorhergehenden Ansprüche, **dadurch gekennzeichnet, dass** die Zubereitung eine dritte Phase umfaßt, wobei die Trägerphase und die zweite Phase mit der weiteren dritten Phase gemischt, die zweite Phase mit der dritten Phase nicht oder nur teilweise mischbar und die dritte Phase vollständig, teilweise oder nicht mit wäßrigen Flüssigkeiten mischbar ist.

14. Zubereitung nach einem oder mehreren der vorhergehenden Ansprüche, **dadurch gekennzeichnet, dass** die zweite Phase natürliche, halbsynthetische oder synthetische Fette, Öle oder Wachse, wie Baumwollensaatöl, Sojaöl, Saffloröl, hydriertes Erdnußöl, Olivenöl, Rizinusöl, Triglyceridgemische (wie Miglyol), Silikonöl, Isopropylmyristat, Ethyloleat, Paraffine, Glycerol, Propylenglycol oder Polyethylenglycol oder Mischungen aus zwei oder mehreren der oben genannten Stoffe umfasst.

15. Zubereitung nach einem oder mehreren der vorhergehenden Ansprüche, **dadurch gekennzeichnet, dass** sie viskositätserhöhende Substanzen enthält und/oder thixotropes Fließverhalten aufweist, dass sie Stabilisatoren, Freigaberegulatoren, Substanzen, welche die Verweildauer am Applikationsort verändern, bioadhäsive Materialien und/oder Penetrationsverbesserer enthält, wobei der Wirkstoff am Applikationsort verzögert freigegeben und/oder eine anfängliche, rasche Freigabe des Wirkstoffes am Applikationsort vermieden wird.

16. Zubereitung nach einem oder mehreren der vorhergehenden Ansprüche zur parenteralen, peroralen, subcutanen, rectalen, buccalen, vaginalen, nasalen, lokalen, sublingualen, peridontalen oder transdermalen Anwendung und/oder zur Einbringung in Körperöffnungen.

17. Zubereitung nach einem oder mehreren der vorhergehenden Ansprüche in Form einer Kapsel.

18. Verfahren zur Herstellung einer Zubereitung nach Anspruch 1, **dadurch gekennzeichnet, dass** man eine Dispersion aus einer flüssigen inneren polymerhaltigen Trägerphase und einer nicht mischbaren oder teilweise nicht mischbaren zweiten äußeren Phase herstellt.

19. Verfahren nach Anspruch 18, **dadurch gekennzeichnet, dass** man die Zubereitung durch Hochdruckhomogenisierung oder unter Erwärmen herstellt, wobei die Teilchengröße der Trägerphase kleiner als 200 µm oder im kolloidalen Bereich liegt.

20. Verfahren nach einem oder mehreren der vorhergehenden Ansprüche 18 oder 19, **dadurch gekennzeichnet, dass** man die Zubereitung kurz vor dem Einbringen in den Körper aus der Trägerphase und der äußeren Phase herstellt.

21. Verfahren nach einem oder mehreren der vorhergehenden Ansprüche 18 bis 20, **dadurch gekennzeichnet, dass** man die Trägerphase und die äußere Phase getrennt voneinander aufbewahrt, die Trägerphase und die äußere Phase in einer Zweikammerspritze aufbewahrt, oder die Trägerphase und die äußere Phase in Kontakt, aber in nicht oder nur teilweise dispergiertem Zustand aufbewahrt.

## Claims

1. An active compound-containing composition, which forms particles after administration in a body, which is obtained through the preparation of a dispersion from a liquid inner polymer-containing carrier phase and a non-miscible or partially non-miscible second external phase and a subsequent change in ambient conditions, wherein the viscosity of the carrier phase changes, and wherein the polymer is selected from a cellulose-derivative, a polyacrylate-derivative, a polysaccharide, a polyanhydride, a polyester, a polyorthoester, a polyurethane, a polycarbonate, a polyphosphazene, a polyacetal or a combination of these polymers.

2. A composition according to claim 1, wherein the change in ambient conditions results in an increase in the concentration of the carrier material in the carrier phase, in a precipitation of the carrier material, in a diffusion of the solvent, wherein the change in ambient conditions is a contact of the composition with body components, a change in temperature, a pH-change, a change in ionic strength or type of ion, a contacting with substances present at the site of administration, or a combination of two or more changes in ambient conditions.

3. A composition according to one or more of the previous claims, which is prepared by high-pressure homogenization.

4. A composition according to one or more of the previous claims, wherein the particle size of the carrier phase is primarily smaller than 100 µm, preferably smaller than 20 µm.

5. A composition according to one or more of the previous claims, wherein the particle size of the carrier phase is primarily in the colloidal size range.

6. A composition according to one or more of the previous claims, wherein the composition is prepared shortly before administration.

7. A composition according to one or more of the previous claims, wherein the carrier phase and the second phase are stored separately from each other or in contact with each other, however, in a non-dispersed or only partially dispersed state.

8. A composition according to one or more of the previous claims, wherein the carrier material is dissolved and/or dispersed in the carrier phase or the carrier material forms the carrier phase by itself.

9. A composition according to one or more of the previous claims, wherein the active compound is a drug, a peptide- or a protein-drug.

10. A composition according to one or more of the previous claims, wherein the active compound is dissolved, dispersed, suspended or emulsified in the carrier phase or is additionally or exclusively present in the other phases.

11. A composition according to one or more of the previous claims, wherein the carrier phase comprises water or an organic solvent, such as ethanol, acetone, butanol, ethylformate, pentanol, n- or i-propanol, tetrahydrofuran, triethylcitrate, triacetin, propylene glycol, glycerol, polyethylene glycol, ethylacetate, methylacetate, dimethylformamide, dimethylsulfoxide, dimethylacetamide, 2-pyrrolidone, N-methyl-2-pyrrolidone or a mixture of two or more of these solvents.

12. A composition according to one or more of the previous claims, wherein the second phase is completely, partially or not miscible with aqueous fluids.

13. A composition according to one or more of the previous claims, wherein it comprises a third phase, wherein the carrier phase and the second phase are mixed with the additional third phase, wherein the second phase is not or only partially miscible with the third phase and wherein the third phase is completely, partially or not miscible with aqueous fluids.

14. A composition according to one or more of the previous claims, wherein the second phase comprises natural, semisynthetic or synthetic lipids, oils or waxes, such as cottonseed oil, soybean oil, safflower oil, hydrated peanut oil, olive oil, castor oil, triglyceride mixtures (like Miglyol), silicone oil, isopropylmyristate, ethyloleate, paraffin, glycerol, propylene glycol or polyethylene glycol or mixtures of two or more of these substances.

15. A composition according to one or more of the previous claims, wherein it comprises viscosity-increasing substances and/or has thixotropic rheological behavior, wherein it comprises stabilizers, release modifying agents, substances, which affect the residence time at the site of administration, bioadhesive materials, penetration enhancers, wherein the active compound is released in an extended fashion at the site of administration and/or wherein an initial rapid release of the active compound at the site of administration is avoided.

16. A composition according to one or more of the previous claims for the parenteral, peroral, subcutaneous, rectal, buccal, vaginal, nasal, local, sublingual, periodontal, or transdermal administration and/or for the placement in body cavities.

17. A composition according to one or more of the previous claims in the form of a capsule.

18. A method of preparing a composition according to claim 1, wherein a dispersion of a liquid inner polymer-containing carrier phase and a non-miscible or partially non-miscible second external phase is prepared.

19. A method according to claim 18, wherein the composition is prepared by high pressure homogenization or under heating, wherein the particle size of the carrier phase is smaller than 200 µm or is in the colloidal size range.

20. A method according to claims 18 or 19, wherein the composition is prepared from the carrier phase and the external phase shortly before administration in the body.

21. A method according to claims 18 or 20, wherein the carrier phase and the external phase are stored separately from each other, wherein the carrier phase and the external phase are stored in a two chamber syringe, or wherein the carrier phase and the external phase are stored in contact but not or only partially in a dispersed state.

## Revendications

1. Une composition, contenant une substance active, qui forme des particules après administration dans le corps, qui est obtenue par la préparation d'une dispersion à partir d'une phase véhicule liquide interne contenant un polymère et d'une deuxième phase externe non miscible ou partiellement non miscible et d'un changement consécutif des conditions ambiantes, **caractérisée en ce que** la viscosité de la phase véhicule change, et **caractérisée en ce que** le polymère est choisi dans le groupe constitué par les dérivés cellulosiques, les dérivés de polyacrylates, les polysaccharides, les polyanhydrides, les polyesters, les polyorthoesters, les polyuréthanes, les polycarbonates, les polyphosphazènes, les polyacétals, ou un mélange de ces polymères.

2. Une composition selon la revendication 1, **caractérisée en ce que** le changement des conditions ambiantes entraîne une augmentation de la concentration du matériel véhicule dans la phase véhicule, une précipitation du matériel véhicule, une diffusion du solvant, le changement des conditions ambiantes est un contact de la composition avec des éléments du corps, un changement de température, un changement de pH, un changement de la force ionique ou du type d'ion, un contact avec des substances présentes au niveau du site d'administration, ou une combinaison de deux changements ou plus des conditions ambiantes.

3. Une composition, selon l'une quelconque des revendications précédentes, qui est préparée par une méthode d'homogénéisation à haute pression.

4. Une composition selon l'une quelconque des revendications précédentes, **caractérisée en ce que** la taille des particules de la phase véhicule est principalement inférieure à 100 µm, de préférence inférieure à 20 µm.

5. Une composition selon l'une quelconque des revendications précédentes, **caractérisée en ce que** la taille des particules de la phase véhicule est principalement dans la classe de grandeurs des colloïdes.

6. Une composition selon l'une quelconque des revendications précédentes, **caractérisée en ce que** la composition est préparée peu de temps avant administration.

7. Une composition selon l'une quelconque des revendications précédentes, **caractérisée en ce que** la phase véhicule et la deuxième phase sont conservées séparément l'une de l'autre ou au contact l'une de l'autre, toutefois, dans un état non dispersé ou seulement partiellement dispersé.

8. Une composition selon l'une quelconque des revendications précédentes, **caractérisée en ce que** le matériel véhicule est dissous et/ou dispersé dans la phase véhicule ou le matériel véhicule forme par lui-même la phase véhicule.

9. Une composition selon l'une quelconque des revendications précédentes, **caractérisée en ce que** la substance active est un principe actif, un peptide-principe actif ou une protéine-principe actif.

10. Une composition selon l'une quelconque des revendications précédentes, **caractérisée en ce que** la substance active est dissoute, dispersée, suspendue ou émulsifiée dans la phase véhicule ou est de plus ou exclusivement présente dans les autres phases.

11. Une composition selon l'une quelconque des revendications précédentes, **caractérisée en ce que** la phase véhicule comprend de l'eau ou un solvant organique tel que l'éthanol, l'acétone, le butanol, l'acide propionique, le pentanol, l'alcool propylique normal ou l'alcool isopropylique, le tétrahydrofurane, le citrate de triéthyle, la triacétine, le propylèneglycol, le glycérol, les polyéthylèneglycols, l'acétate d'éthyle, l'acétate de méthyle, le diméthylformamide, le diméthylsulfoxyde, le diméthylacétamide, le 2-pyrrolidone, le N-Méthyl-2-pyrrolidone ou les mélanges de deux ou plus de ces solvants.

12. Une composition selon l'une quelconque des revendications précédentes, **caractérisée en ce que** la deuxième phase est complètement, partiellement ou non miscible avec les liquides aqueux.

13. Une composition selon l'une quelconque des revendications précédentes, **caractérisée en ce que** la composition comprend une troisième phase, **caractérisée en ce que** la phase véhicule et la deuxième phase sont mélangées avec la troisième phase additionnelle, **caractérisée en ce que** la deuxième phase est non miscible ou seulement partiellement miscible avec la troisième phase, et **caractérisée en ce que** la troisième phase est complètement, partiellement ou non miscible avec les liquides aqueux.

14. Une composition selon l'une quelconque des revendications précédentes, **caractérisée en ce que** la deuxième phase comprend des lipides naturels, semi-synthétiques ou synthétiques, des huiles ou des cires, comme l'huile de coton, l'huile de soja, l'huile de carthame, l'huile d'arachide hydrogénée, l'huile d'olive, l'huile de ricin, les mélanges de triglycérides (comme le Miglyol), l'huile de silicone, le myristate d'isopropyle, l'oléate d'éthyle, la paraffine, le glycérol, le propylèneglycol ou les polyéthylèneglycols ou les mélanges de deux ou plus de ces substances.

15. Une composition selon l'une quelconque des revendications précédentes, **caractérisée en ce que** elle comprend des substances augmentant la viscosité et/ou a un comportement rhéologique thixotropique, **caractérisée en ce qu'**elle comprend des agents stabilisants, des agents modifiant la libération, des substances qui modifient le temps de résidence au niveau du site d'administration, des matériaux bio-adhésifs, des promoteurs d'absorption, **caractérisée en ce que** la substance active est libérée de façon prolongée au niveau du site d'administration et/ou **caractérisée en ce que** une libération initiale rapide de la substance active au niveau du site d'administration est évitée.

16. Une composition selon l'une quelconque des revendications précédentes pour l'administration parentérale, per os, sous-cutanée, rectale, buccale, vaginale, nasale, locale, sublinguale, périodontale ou transdermique et/ou pour le placement dans des cavités du corps.

17. Une composition selon l'une quelconque des revendications précédentes, sous forme d'une capsule.

18. Une méthode de préparation de composition selon la revendication 1, **caractérisée en ce que** une dispersion d'une phase véhicule liquide interne contenant un polymère et d'une deuxième phase externe non miscible ou partiellement miscible est préparée.

19. Une méthode selon la revendication 18, **caractérisée en ce que** la composition est préparée par une méthode d'homogénéisation à haute pression ou par chauffage, **caractérisée en ce que** la taille des particules de la phase véhicule est inférieure à 200 µm ou est dans la classe de grandeurs des colloïdes.

20. Une méthode selon les revendications 18 ou 19, **caractérisée en ce que** la composition est préparée à partir d'une phase véhicule et d'une phase externe peu de temps avant l'administration dans le corps.

21. Une méthode selon les revendications 18 ou 20, **caractérisée en ce que** la phase véhicule et la phase externe sont conservées séparément l'une de l'autre, **caractérisée en ce que** la phase véhicule et la phase externe sont conservées dans une seringue à deux compartiments, ou **caractérisée en ce que** la phase véhicule et la phase externe sont conservées au contact l'une de l'autre mais pas dans un état dispersé ou dans un état dispersé seulement partiellement.
